# EUROPEAN PATENT SPECIFICATION

(11) **EP 1 173 764 B1**
(45) Date of publication and mention of the grant of the patent: **01.03.2006**
(21) Application number: 00922683.8
(22) Date of filing: 28.04.2000
(51) Int. Cl.: G01N 33/573, G01N 33/74

(54) **METHOD FOR IDENTIFYING AN INDIVIDUAL AT RISK FOR IRREVERSIBLE NEURODAMAGES, COMPRISING THE STEPS OF DETERMINING QUANTITATIVELY THE CONCENTRATION OF PEPSINOGEN I (PGI) AND VITAMIN B12**
VERFAHREN ZUR ABSCHÄTZUNG DES INDIVIDUELLEN RISIKOS EINER IRREVERSIBLEN NERVENSCHÄDIGUNG, DAS SCHRITTE ZUR QUANTITATIVEN BESTIMMUNG DER PEPSINOGEN-I (PGI) UND VITAMIN B12 KONZENTRATIONEN UMFASST
PROCEDE D'IDENTIFICATION D'UN INDIVIDU PRESENTANT LE RISQUE DE DEVELOPPER DES ALTERATIONS NEUROLOGIQUES IRREVERSIBLES CONSISTANT A DETERMINER DE MANIERE QUANTITATIVE LA TENEUR EN PEPSINOGENE I (PGI) ET EN VITAMINE B12

(30) Priority: 30.04.1999 FI 990991
(43) Date of publication of application: 23.01.2002
(73) Proprietor: BIOHIT OY, 00880 Helsinki (FI)
(72) Inventor: SIPPONEN, Pentti, FIN-02160 Espoo (FI); HÄRKÖNEN, Matti, FIN-02110 Espoo (FI); SUOVANIEMI, Osmo, FIN-00570 Helsinki (FI); FORSBLOM, Erik, FIN-02360 Espoo (FI)
(74) Representative: Grew, Eva Regina
(86) International application number: PCT/FI2000/000376
(87) International publication number: WO 2000/067029

(56) References cited:
- WO-A1-96/15456
- B. REGLAND ET AL.: 'Dementia patients with low serum cobalamin concentration: Relationship to atrophic gastritis' AGING CLIN. EXP. RES. vol. 4, no. 1, 1992, pages 35 - 41, XP000965073
- A. LINDGREN ET AL.: 'Advantages of serum pepsinogen A combined with gastrin or pepsinogen C as first-line analytes in the evaluation of suspected cobalamin deficiency: a study in patients previously not subjected to gastrointestinal surgery' JOURNAL OF INTERNAL MEDICINE vol. 244, 1998, pages 341 - 349, XP002933115

## Description

### Field of the invention

The present invention relates to a combination diagnostic method for identifying individuals who are at risk for irreversible neurological damages, including dementia.

The method according to the invention is based on the combination of two tests carried out on a blood or serum sample especially in order to identify patients prone to develop a B12 deficiency and thus at risk of developing afflictions resulting from such deficiency, such as irreversible neurological damages, including dementia and pernicious anaemia.

### Background of the invention

A number of different gastric diseases or conditions, such as chronic atrophic gastritis, pernicious anaemia, ventricular ulcer, gastric polyposis and the Ménétrier disease (giant hypertrophic gastritis) precede gastric cancer. Clearly identifiable changes of the mucosa are dysplasia and adenoma. It has been established that in almost all diseases the risk is mediated over chronic atrophic gastritis.

Chronic gastritis means a prolonged inflammatory condition of the gastric mucosa. The disease can coarsely be divided into the superficial and the atrophic form. In superficial gastritis, the inflammatory cell infiltration is concentrated below the surface epithelium. In case the inflammation progresses and diffuses between the specific gastric secretory glands, one refers to chronic atrophic gastritis. In such a case, the normal glandular structures of the gastric mucosa are at least partly substituted by metaplastic changes.

The relative risk of gastric cancer in patients suffering from atrophic gastritis in the corpus area of the stomach has been estimated, as calculated from the Finnish cancer statistics, to be about 4- to 5-fold as compared to persons having a healthy mucosa. In addition, there is a risk for falling ill with pernicious anaemia due to intrinsic factor deficiency and B12 vitamin absorption disturbance. In severe atrophy of the antrum area, the risk is even 18-fold. If atrophic changes appear both in the antrum and the corpus area (pangastritis), the risk can increase co even 90-fold.

The publication WO 96/15456 discloses a method for screening for the risk of gastric cancer according to which atrophy in the mucosa either of the corpus or the antrum area, or of both, is determined by determining the pepsinogen I (PGI) and gastrin-17 (G-17) analyte levels in a serum sample, and comparing the levels so determined to a method-specific cut-off value for respective analyte. The levels determined are preferably also compared to a method-specific reference value for respective analyte.

The publication B. Regland, Aging Clin. Exp. Res. Vol. 4, no. 1, 1992, p. 35-41, relates to a study assessing a relationship between the serum concentration of PGI and gastrin on the one hand and B12 vitamin on the other hand, in patients with dementia disorders.

A serum PGI value below the specific cut-off value for PGI indicates atrophic gastritis in the corpus area of the stomach. If the serum G-17 concentration is below its cut-off value, the atrophy is located in the antrum area of the stomach. In pangastritis, the serum PGI is below the cut-off value and the serum G-17 value is at the lower limit of its reference value.

The supply of sufficient B12 vitamin is necessary for folate metabolism and normal blood production, as well as for the function of the nerve cells. Vitamin B12 forms a complex with a protein, the intrinsic factor, produced by the mucosa of the corpus area of the stomach, which complex is resorbed in the lower part of the ileum. This is the preferred resorption form of vitamin B12.

A deficiency of intrinsic factor, as a result of atrophic gastritis or stomach cancer, especially in the corpus area of the stomach, will ultimately lead to a deficiency in B12 vitamin in the body. A prolonged deficiency can lead to the development of pernicious anaemia, and even earlier, within one year, to irreversible neurological damage and dementia. A B12 vitamin deficiency is thus an important risk factor to consider in the development of neurological disorders, such as dementia, especially in the older population. It has been estimated that a stealthy shortage of B12 vitamin afflicts hundreds of thousands of people. In these cases, treatment with B12 vitamin injections would be simple and effective, if the shortage was known. It would be especially valuable to be able to identify those individuals which have not yet developed B12 vitamin deficiency but which are at a substantial risk of doing so in the future, unless measures are taken to prevent such deficiency from developing.

### Summary of the invention

The present invention is directed to a method for combining an assay of determining, in a serum sample, a marker for atrophic gastritis in combination with an assay for vitamin B12, in order to assist in the diagnosis of, or in the determination of the risk of, irreversible, neurological damage in an individual.

The method according to the invention is a method for identifying an individual at risk for irreversible neurological damage and pernicious anaemia, the method comprising the steps of
- determining quantitatively the pepsinogen I (PGI) analyte concentration in a serum sample from said individual,
- selecting a method specific cut-off value for the said analyte,
- comparing the analyte concentration so determined to the method-specific cut-off value for the analyte, and
- determining the concentration of an analyte selected from vitamin B12, methylmalonate and homocysteine, in a serum sample from the said individual and comparing it to a method-specific reference value for the analyte so determined, whereby a serum PGI concentration value below its specific cut-off value in combination with a normal vitamin B12, methylmalonate or homocysteine concentration value, is an indication bf such a risk.

The determination of the serum PGI and vitamin B12 concentrations, or of methylmalonate or homocysteine correlating to the B12 concentration, can take place in any order, in order to simultaneously obtain knowledge of both the serum PGI and vitamin B12 levels for the purpose of assisting in the diagnosis of, or assessing the risk of neurological damage in said individual. However, according to an embodiment of the invetion, the serum PGI concentration is determined and compared to its method-specific cut-off value, and for further diagnosis an individual is selected who has a serum PGI concentration value below its method-specific cut-off value. In this embodiment, only the individuals which exhibit low serum PGI values indicative of corpus atrophy, are screened for vitamin B12.

Thus according to a preferred embodiment, the present invention is especially aimed at screening such individuals, which do not yet exhibit a B12 vitamin deficiency, that is which exhibit substantially normal B12 vitamin levels, but which due to diagnosed atrophy in the corpus area of the stomach are prone to develop such deficiency over time. Such an identification makes it possible to already at an early stage resort to preventative measures in order to counteract the development of the afflictions associated with B12 vitamin deficiency, especially irreversible neurological damage which develops earlier than anaemia.

### Detailed description of the invention

### 1. Determination of pepsinogen I (PGI)

The method of determining PGI in a serum sample can be carried out as is described in the publication WO 96/15456, which publication is included herein for reference.

The said method preferably includes the steps of using poly- or monoclonal antibodies to pepsinogen I. in an immunological method for the determination of pepsinogen I. Suitably the reaction is carried out on a suitable support, such as a plastic, glass or cellulose support, for example on a microplate. The immunological methods can be carried out in a known manner, using e.g. absorbance, luminescence or fluoresence techniques for measuring the said pepsinogen I concentration in the sample.

The method specific cut-off value, in this case for serum PGI, is selected depending on the specificity and sensitivity chosen for the test used for the determination of the analyte concentration, and is as such well known to the person skilled in the art, see e.g. William J Marshall, Clinical Chemistry, Third Edition, 1995, Mosby, especially page 7.

If the serum pepsinogen I concentration is below the cut-off value, which depending on the specificity and sensitivity agreed upon for the method in question, is 20-30 µg/l, which corresponds to appr. 450 - 690 pmol/l, there is atrophy in the corpus area of the stomach. The normal or reference value for PGI is in the range of 25 - 120 µg/l.

### 2. Determination of B12-vitamin

The B12-vitamin (cobalamin) concentration in a serum sample can be determined according to any of the methods *per se* known for this purpose. Such known methods include microbiological assay of serum B12 employing an organism, such as *Euglena gracilis* or *Lactobacillus leichmannii* which requires cobalamin for growth. Also radioisotope dilution assays for B12 vitamin have been utilized and such assay techniques are well documented in the literature, e.g. Lau *et al*., "Measurement of serum B12 levels using Radioisotope Dilution and Coated Charcoal", Blood, 26 (1965), 202. Radioisotope dilution methods are more rapid and give results comparable with those of e.g. the *Euglena* assay, provided the binding protein is specific for biologically active cobalamin. A standardized pure or purified intrinsic factor preparation is most satisfactory as the binding protein as it binds specifically to true cobalamin rather than cobalamin analogues.

The radioisotope dilution assay of B12 generally includes the step of freeing endogenous B12 from its natural binding protein e.g by boiling at a selected pH and then adding a measured amount of the radioisotope 57Co-B12, and a limited amount of binding protein. All of the binding protein will be bound by some form of B12 since the amount of radioisotope B12 added is sufficient to bind the small amount of protein. As both the natural and the radioactive B12 compete to bind with the binding protein, the degree to which the radioactive count of the protein bound B12 was inhibited is indicative of the amount of B12 in the sample. This method has been modified by Lau, *supra,* by separating unbound B12 from protein bound B12 by protein coated charcoal and the radioactivity of the supernatant liquid containing the mixture of bound radioactive B12 and bound non-radioactive B12 is counted for radioactivity. The serum B12 concentration is then calculated from the count, often by comparison with a standard chart. Radioassay kits are commercially available for carrying out the method.

There are also other methods available for assaying B12 levels in a serum sample. Because a cobalamin coenzyme is essential for the isomerization of methylmalonate to succinate, excretion of increased amounts of methylmalonate is found in B12 vitamin deficiency. This provides a sensitive test for B12 vitamin deficiency. In addition plasma levels of homocysteine are increased as well. The US patent 4,940,658 describes a method for detecting elevated levels of homocysteine, by combining a sample with a known amount of labelled homocysteine, and determining the ratio of labelled to unlabelled homocysteine, e.g. by mass-spectrographic methods, and then calculating the amount of unlabelled homocysteine in the sample.

B12 vitamin deficiency has also been determined using e.g. chemiluminiscence receptor assays (Wentworth, S. *et al*., Clin. Chem., vol 40 537-540), radioimmunoassays (Endres, D.B., *et. al.* Clin. Chem., Vol. 24, 460-465) as well as nonisotopic binding assays, CEDIA, cloned enzyme donor immunoassays (van der Weide, J. *et al*. Clin. Chem., Vol. 38, 766-768).

Any of the known methods and kits available for determining the concentration of B12, or of methylmalonate or homocysteine, an increased concentration of which can be taken as being indicative of B12 deficiency in the sample, can be used in the method according to the invention. The invention also contemplates the possibility of determining both the B12 concentration and that of the analyte correlating to the B12 concentration.

According to the invention, the B12 vitamin concentration measured is compared to a selected method specific reference value or range for B12. Such a reference value varies between 200-900 ng/l, corresponding to appr. 170 to 700 pmol/l. As regards homocysteine, a reference value of less than 15 - 16 µmol/l., and of methylmalonate a reference value of less than 0.4 µmol/l. have been recommended.

## Claims

1. Method for identifying an individual at risk for irreversible neurological damage and pernicious anaemia, the method comprising the steps of
- determining quantitatively the pepsinogen I (PGI) analyte concentration in a serum sample from said individual,
- selecting a method specific cut-off value for the said analyte,
- comparing the analyte concentration so determined to the method-specific cut-off value for the analyte, and
- determining the concentration of an analyte selected from vitamin B12, methylmalonate and homocysteine, in a serum sample from the said individual, and comparing it to a method-specific reference value for the analyte so determined, whereby a serum PGI concentration value below its specific cut-off value in combination with a normal vitamin B12, methylmalonate or homocysteine concentration value, is an indication of such a risk.

2. The method according to claim 1, comprising determining the serum PGI concentration, and selecting for further determination of the concentration of B12 vitamin or of methylmalonate or homocysteine, an individual who exhibits a serum PGI concentration below the cut-off value for PGI.

3. The method according to claim 1, wherein the B12 vitamin concentration in the sample is determined.

## Patentansprüche

1. Verfahren zum Identifizieren eines Individuums, bei dem das Risiko von irreversiblem neurologischem Schaden und perniziöser Anämie besteht, wobei das Verfahren die Schritte umfasst:
- quantitatives Bestimmen der Pepsinogen I (PGI)-Analytkonzentration in einer Serumprobe des Individuums,
- Auswählen eines verfahrensspezifischen Cut-Off-Wertes für den Analyt,
- Vergleichen der so bestimmten Analytkonzentration mit dem verfahrensspezifischen Cut-Off-Wert für den Analyt, und
- Bestimmen der Konzentration eines Analyts, ausgewählt aus Vitamin B12, Methylmalonat und Homocystein, in einer Serumprobe des Individuums, und Vergleichen dieser mit einem verfahrensspezifischen Referenzwert für den Analyt, der so bestimmt wurde, wobei ein Serum-PGI-Konzentrationswert unterhalb seines spezifischen Cut-Off-Werts in Kombination mit einem normalen Vitamin B 12-, Methylmalonat- oder Homocystein-Konzentrationswert eine Indikation für ein solches Risiko darstellt.

2. Verfahren gemäß Anspruch 1, umfassend Bestimmen der Serum-PGI-Konzentration und Auswählen eines Individuums, das eine Serum-PGI-Konzentration unterhalb des Cut-Off-Werts für PGI aufweist, zur weiteren Bestimmung der Konzentration von Vitamin B12 oder von Methylmalonat oder Homocystein.

3. Verfahren gemäß Anspruch 1, wobei die Vitamin B12-Konzentration in der Probe bestimmt wird.

## Revendications

1. Procédé d'identification d'un individu présentant le risque de développer des altérations neurologiques irréversibles et une anémie pernicieuse, le procédé comprenant les étapes consistant à :
- déterminer de manière quantitative la teneur en substance à analyser de pepsinogène I (PGI) dans un échantillon de sérum dudit individu,
- choisir pour ladite substance à analyser une valeur de coupure spécifique au procédé,
- comparer la teneur en substance à analyser ainsi déterminée à la valeur de coupure spécifique au procédé pour la substance à analyser, et
- déterminer la teneur d'une substance à analyser choisie dans le groupe comprenant la vitamine B12, le méthylmalonate et l'homocystéine, dans un échantillon de sérum provenant dudit individu et la comparer à la valeur de référence spécifique au procédé pour la substance à analyser ainsi déterminée, moyennant quoi une valeur de teneur en PGI du sérum inférieure à sa valeur de coupure spécifique, en combinaison avec des valeurs de teneur en vitamine B12, méthylmalonate ou homocystéine normales, est une indication d'un tel risque.

2. Procédé selon la revendication 1, comprenant la détermination de la teneur en PGI du sérum et le choix, pour une détermination ultérieure de sa teneur, entre la vitamine B12, le méthylmalonate et l'homocystéine, chez un individu présentant une teneur en PGI du sérum inférieure à la valeur de coupure pour le PGI.

3. Procédé selon la revendication 1, dans lequel la teneur en vitamine B12 dans l'échantillon est déterminée.
